(19) Europäisches Patentamt
European Patent Office
Office européen des brevets

(11) **EP 3 638 338 B1**

(12) **EUROPEAN PATENT SPECIFICATION**

(45) Date of publication and mention
of the grant of the patent:
**28.08.2024 Bulletin 2024/35**

(21) Application number: **18731390.3**

(22) Date of filing: **08.06.2018**

(51) International Patent Classification (IPC):
**A61M 1/28** *(2006.01)*     **A61M 1/16** *(2006.01)*
**G16H 20/40** *(2018.01)*     **G16H 10/00** *(2018.01)*

(52) Cooperative Patent Classification (CPC):
**A61M 1/28; A61M 1/1613; G16H 10/60;
G16H 20/40; G16H 50/50;** A61M 2205/52

(86) International application number:
**PCT/EP2018/065163**

(87) International publication number:
**WO 2018/228942 (20.12.2018 Gazette 2018/51)**

(54) **METHOD AND APPARATUS FOR GENERATING TREATMENT REGIMENS**

VERFAHREN UND VORRICHTUNG ZUR ERZEUGUNG EINES BEHANDLUNGSPLANS

PROCÉDÉ ET APPAREIL POUR GÉNÉRER DES RÉGIMES DE TRAITEMENT / SCHÉMAS THÉRAPEUTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.06.2017 RO 201700362**

(43) Date of publication of application:
**22.04.2020 Bulletin 2020/17**

(73) Proprietor: **Fresenius Medical Care Deutschland
GmbH
61352 Bad Homburg (DE)**

(72) Inventors:
  • **MUSCA, Gheorghe**
    **Bucharest (RO)**
  • **POPESCU, Ariana**
    **Bucharest (RO)**

(74) Representative: **Vasilescu, Raluca
    Cabinet M. Oproiu
    Patent and Trademark Attorneys
    42, Popa Savu Street
    Sector 1, P.O. Box 2-229
    011434 Bucharest (RO)**

(56) References cited:
**WO-A2-2010/006131     US-A1- 2011 009 810**

  • **DONATA VAITKUNAITE ET AL: "Adapted
    Automated Peritoneal Dialysis: Importance of
    Varying Dwell Time and Fill Volume", MEDICINOS
    TEORIJA IR PRAKTIKA, vol. 20, no. 3, 25 July 2014
    (2014-07-25), pages 217 - 220, XP055494060,
    ISSN: 1392-1312, DOI: 10.15591/mtp.2014.035**
  • **MICHEL FISCHBACH ET AL: "Optimizing
    peritoneal dialysis prescription for volume
    control: the importance of varying dwell time and
    dwell volume", PEDIATRIC NEPHROLOGY., vol.
    29, no. 8, 1 August 2014 (2014-08-01), DE, pages
    1321 - 1327, XP055494069, ISSN: 0931-041X, DOI:
    10.1007/s00467-013-2573-x**
  • **MICHEL FISCHBACH: "Adapted Automated
    Peritoneal Dialysis", ADVANCES IN
    PERITONEAL DIALYSIS, 1 January 2014
    (2014-01-01), XP055494074, Retrieved from the
    Internet
    <URL:https://pdfs.semanticscholar.org/88fa/8a4
    efa7aafe084c7c89d4a0aa98f30a86456.pdf>
    [retrieved on 20180720]**

EP 3 638 338 B1

## Description

The present invention relates to the field of generating a treatment regimen, in particular for generating a regimen for operating a peritoneal dialysis treatment apparatus.

## Background

The proportion of patients performing automated peritoneal dialysis ("APD") is increasing worldwide, which is due in part to the ability of APD to be adapted to the patient's particular needs regarding the patient's private life and the patient's therapy needs. The two primary goals of dialysis, solute clearance and ultrafiltration ("UF") depend on the modality or type of APD performed

Historically, APD devices typically did not have the capability to provide feedback to the patient regarding the effectiveness of his/her recent therapies. Also, APD devices typically ran open loop such that they do not adjust therapy parameters (e.g., modality, solution type, therapy time and fill volume) based on the actual measured daily clearance and UF. Accordingly, a substantial risk existed, that some patients underachieved their targets and developed adverse conditions such as fluid overload and in some cases hypertension.

To address the above problems computerised prescription optimization systems have been developed that use patient physiological data in combination with specified therapy targets (e.g., ultrafiltration volume, clearance) and ranges of therapy inputs (e.g., number of exchanges, inflow volumes, cycle times, solution choices) to calculate or deliver possible regimens that fit within the provided ranges.

In US2011/0009810A1 is described a method of calculating a peritoneal dialysis therapy, comprising inputting data of pacient, selecting a fill volume for a cycle of the peritoneal dialysis therapy, selecting at least one therapy parameter or at least one desired outcome of the peritoneal dialysis therapy, and calculating a therapy for the pacient using a digital computer, wherein the therapy is calculated without regard to values for at least one of the therapy volume and a cycle time.

Patient characteristics include mass transfer area coefficient ("MTAC") data and hydraulic permeability data to classify the patient's transport and UF characteristics.

To simulate the outcome of a peritoneal dialysis treatment various algorithms have been developed and described in the literature [Kinetic modelling in Peritoneal Dialysis, Gotch, Keen, Clinical Dialysis, 4th edition, 2005: 385- 419]. One algorithm frequently used to simulate a dialysis treatment outcome employs a three-pore model, which adds predicted clearances across three different sized pores in the patient's peritoneal membrane. Flow of toxins through large pores, small pores and micropores in the blood vessels of the peritoneal membrane are summed. Urea and creatinine for example are removed from the patient's blood through the small pores. The large pores allow larger protein molecules to pass from the patient's blood to the dialysate. In each case, an osmotic gradient drives the toxins from the patient's blood to the dialysate. In a more simplified two pore model only two different sized pores are considered. Alternatively a so called two-pool peritoneal dialysis kinetic model can be used to predict fluid and solute removal in peritoneal dialysis to simulate a therapy outcome. The two pool model utilizes a set of differential equations that collectively describe both diffusive and convective mass transport in both the body and dialysate compartments for an "equivalent" membrane core.

A prescription optimization system typically applies a two pore, three pore or two pool model or other model to use target information and other therapy input information to generate the regimens.

A dialysis system having regimen generation methodology is described in the international patent application published as WO 2010/006131 A2, wherein is configured a peritoneal dialysis system including a logic implementer capable to provide a plurality of prescription optimization modules.

The inventors of the present application have found out that conventional therapy regimens not always provide optimum treatment outcome, resulting in patients underachieving their therapy objectives.

The problem to be solved by the present invention is providing multiple regimen candidates available for selection of the regimen best adapted for each patient given the patient characteristics input and the therapy target input, and to provide an improved computer-implemented method using a computer program for generating a peritoneal dialysis regimen for controlling peritoneal dialysis treatment in view of an improved achievement of therapy objectives and a corresponding device.

## Summary

The above-mentioned object is solved by a computer-implemented method of generating a peritoneal dialysis regimen for controlling the operation of a peritoneal dialysis treatment machine to meet a therapy target or therapy target range. The computer-implemented method comprises:

> receiving a therapy target input,
> receiving a patient characteristics input,
> generating a plurality of peritoneal dialysis treatment regimens, each peritoneal dialysis regimen being defined by a succession of first cycles and second cycles and [by a corresponding number of first cycles and a number of second cycles],
> simulating a peritoneal dialysis treatment outcome for each of the plurality of dialysis treatment regimens considering the inputted patient characteristics to generate a simulated treatment outcome, and making a peritoneal dialysis treatment regimen to be applied for a specific treatment available for selection based on the simulated treatment outcome and the inputted therapy target.

According to the proposed computer-implemented method the first cycles are defined by a first inflow volume, a first dwell time and the second cycles are defined by a second inflow volume, a second dwell time and the number of first cycles and/or the number of second cycles are variable and/or independent from each other among the plurality of generated peritoneal dialysis treatment regimens.

Advantageously, the computer-implemented method comprises receiving a treatment parameter range input, describing an allowable range for one or more treatment parameters or in other words a set of one or more treatment restrictions and wherein the plurality of peritoneal dialysis regimes are generated within the allowable range of the one or more treatment parameters.

Further advantageously, a first glucose concentration is defined for the first cycles, and a second glucose concentration is defined for the second cycles, the second concentration being different from the first concentration.

Furthermore, the above mentioned object is solved by the subject matter of the independent claims. Advantageous embodiments are provided by the dependent claims.

**Brief description of the drawings**

Fig. 1 depicts a computer-implemented method of generating a peritoneal dialysis treatment regimen and applying the generated treatment to a patient Fig. 2 depicts a peritoneal dialysis treatment regimen generation device

Fig.3 depicts a peritoneal dialysis machine including a treatment regimen generation device

Fig. 4 depicts a peritoneal dialysis system including a database assembly and a peritoneal dialysis machine

Figs. 5-10 depict graphical representations of candidate sets of peritoneal dialysis treatment regimens

**Detailed description of the drawings**

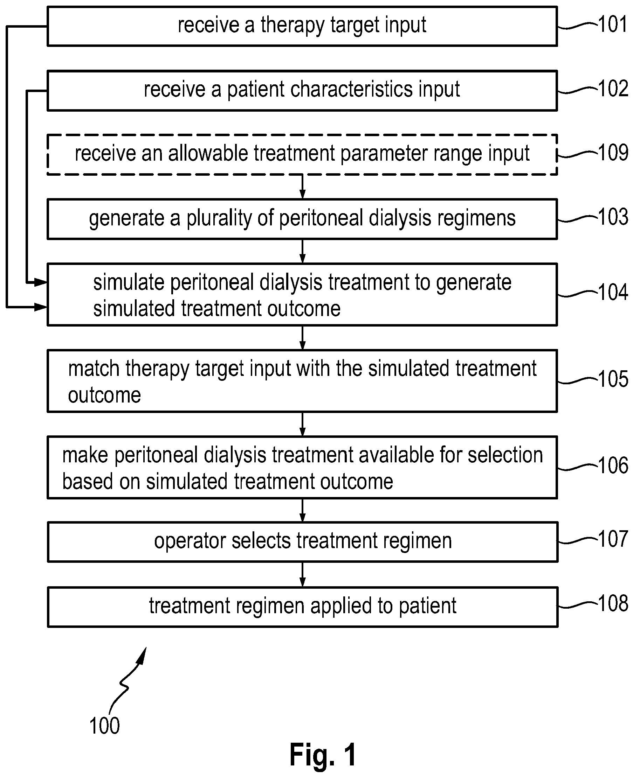

**Fig.1** depicts an embodiment of the computer-implemented method 100 of generating a peritoneal regimen for controlling the operation of a peritoneal dialysis treatment machine to perform a peritoneal dialysis treatment.

Each peritoneal dialysis regimen defines a peritoneal dialysis treatment in terms of the peritoneal dialysis solution applied for the treatment and thereby the glucose concentration, and by the number, fill volume and timing of the sequence of exchange cycles, the timing including a filling time, a dwell time and a drain time.

The computer-implemented method comprises an inputting step 101 of receiving a therapy target input or therapy target range input, in particular a peritoneal dialysis therapy target or target range input from a patient database assembly or from an operator such as a patient, nurse, physician, caregiver or person assisting the patient in supervising his or her therapy. A therapy target value defines a desired therapy outcome in terms of one or more parameter values describing the therapy outcome. The therapy target value may be prescribed by a physician. In one embodiment a therapy target is defined by an ultrafiltration volume or a solute clearance such as a creatinine or urea clearance or by a combination of the above.

Advantageously, the computer-implemented method comprises an inputting step 109 of receiving a treatment parameter range input from a database assembly or from an operator. The treatment parameter range describes an allowable range for one or more treatment parameters or in other words a set of one or more treatment restrictions. The treatment parameter range may be associated with a particular patient or a particular peritoneal dialysis machine or machine type.

E.g. a therapy restriction may define a certain safety parameter like maximum inflow volume allowed for a specific patient and/or a certain quality of life specific parameter like a maximum total therapy time. APD patients often have to end the night APD treatment at a certain defined time in the morning, and therefore the total therapy time has to be limited.

The computer-implemented method comprises a further inputting step 102 of receiving a patient characteristics input such as a peritoneum transport characteristics or BSA (body surface area) or other patient characteristics from a database assembly or from an operator. The body surface area of the patient may be estimated based on an epidemiological equation such as the Du Bois equation using the height and the weight of the patient as input. The peritoneum transport characteristics of the patient, which is sometimes termed as transporter type may be previously determined by a peritoneal function test. For certain predefined equilibrium conditions, a particular D/P ratio (dialysate over plasma ratio) for creatinine, or possibly urea may be regarded as corresponding to a particular transporter type.

In a regimen generation step 103 a set of peritoneal dialysis regimens within a parameter range or "solution space", e.g. within the previously inputted allowable treatment parameter range/ therapy restriction(s) is generated, each peritoneal dialysis regimen defined by a succession of exchange cycles, which in turn are defined by inflow volume, i.e. the volume of peritoneal dialysis solution initially infused in to the patient, dwell time, i.e. the time during which solution is neither actively withdrawn or infused, and total exchange time which is the sum of infusion time, dwell time and withdrawal or drain time. Furthermore a peritoneal dialysis regimen may also include a description of the peritoneal dialysis solution to be applied for a certain treatment, including a value describing the glucose concentration of the solution.

In accordance with the teachings of the present invention each peritoneal dialysis regimen is defined by a succession of first cycles and second cycles, the first cycles of each of the plurality of peritoneal dialysis regimens being defined by a first inflow volume, a first dwell time and the second cycles of each of the plurality of peritoneal dialysis regimens being defined by a second inflow volume, a second dwell time wherein each treatment regimen is defined by a number of first cycles and by a number of second cycles, the number of first cycles and/ or the number of second cycles is variable and/ or independent from each other among the plurality of generated peritoneal dialysis treatment regimens.

In one embodiment the first cycles are shorter and smaller than the second cycles. In this embodiment, the inflow volume of the first i.e. smaller cycles may be determined according to a first predefined relation to the body surface area such as 800ml/m^2 BSA of the patient. The dwell time and total cycle time of the shorter cycles may be each determined according to a corresponding predefined relation to the transporter type or peritoneum transport characteristics for the shorter cycles. The inflow volume of the larger cycles may be determined according to a second predefined relation to the body surface area of the patient such as 1500ml/ m^2 BSA (body surface area) of the patient. The dwell time and total cycle time of the longer cycles may be each determined according to a corresponding predefined relation to the peritoneum transport characteristics or transporter type for the longer cycles. The shorter and smaller cycles precede the longer and larger cycles. In accordance with this embodiment the number of shorter and smaller/ shorter cycles is a first degree of freedom and the number of larger/longer cycles is a further degree of freedom within the solution space or parameter range for peritoneal dialysis treatment regimens. In this embodiment the number of first cycles is independent from the number of second cycles and both the number of first cycles and the number of second cycles are variable.

Another degree of freedom within the parameter range or solution space of peritoneal dialysis treatment regimens may be the peritoneal dialysis solution type and/or glucose concentration of the peritoneal dialysis solution to be applied. The parameter range of the glucose concentration may be sampled depending on the demands of the subsequent simulation step and/ or depending on available peritoneal dialysis solutions as well as based on the capabilities of the PD machine. In one embodiment the PD machine may be provided with a capability to provide glucose profiling, i.e. the capability to adapt the glucose concentration during treatment. In this context a first glucose concentration may be defined for the first cycles and a second glucose concentration may be defined for the second cycles. According to another embodiment the solution type and/or glucose concentration of the peritoneal dialysis solution to be applied may be predefined.

In accordance with a first variation of the regimen generation step 103, a fixed ratio is defined between the size of exchange volumes of small exchange cycles and the size of exchange volume of large exchange cycles. In accordance with this first variation, the absolute volume of either the small or the large exchange cycle is an additional degree of freedom. Thus, the parameter range or solution space has the following degrees of freedom: the absolute size of either the small or the large exchange cycles, the number of small cycles, the number of large cycles, and the solution type /glucose concentration of the peritoneal dialysis solution, or the first and the second glucose concentration of the peritoneal dialysis solution described above. The parameter range may be sampled depending on the demands of the subsequent simulation step and/ or, in the case of the glucose concentration depending on available peritoneal dialysis solutions, and/or PD machine capabilities. In accordance with a second variation of the above described regimen generation step 103, the dwell times and total cycle times for the shorter and for the longer cycles are calculated according to a respective predefined relation to the transport characteristics or transporter type. In accordance with the second variation, the absolute volume of the small and the large exchange cycle are additional degrees of freedom. Thus, the parameter range or solution space has the following degrees of freedom: the absolute size of the small and the large exchange cycle, the number of small cycles, the number of large cycles, and the solution type/ glucose concentration of the peritoneal dialysis solution , or the first and the second glucose concentration of the peritoneal dialysis solution described above. The parameter range may be sampled according to the demands of the subsequent simulation step, and/or available peritoneal dialysis solutions, and/or PD machine capabilities. (for the glucose concentration).

In accordance with a further variation of the above described regimen generation step 103, the dwell times of the larger and the smaller exchange cycles are additional degrees of freedom. Furthermore, like in the second variation the absolute volumes of the small respectively the large exchange cycle are variable. Thus, the solution space or parameter range has the following degrees of freedom: the dwell time of the small and of the large exchange cycles, the absolute size of the small and the large exchange cycle, the number of small cycles, the number of large cycles, and the glucose concentration of the peritoneal dialysis solution, or the first and the second glucose concentration of the peritoneal dialysis solution described above. In a simulation step 104 the prescription parameters are varied within the predefined (allowable) treatment parameter range or solution space, and inputted into a simulation tool to determine a simulated treatment outcome or quality parameter. In one embodiment the simulated treatment outcome is expressed in terms of ultrafiltration, creatinine or urea clearance or in terms of a cost function combining these parameters.

For the simulation of the treatment outcome a three pore model, two pore model or two pool model may be applied as is known in the art.

In a matching step 105 the previously input therapy target or therapy target range is matched with the simulated treatment outcome for each of the peritoneal dialysis treatment regimens within the set of peritoneal dialysis treatment regimens to generate a set of prescription candidates, that meet the predefined allowable treatment target or treatment target range, or in other words, the prescriptions of the set of prescription candidates within the (allowable) treatment parameter range having a simulated treatment outcome within the allowable treatment outcome range.

The prescriptions within the set of treatment regimen candidates are made available for selection by an operator in a step 106.

The operator may then select the prescription to be applied for a certain treatment within the set of selectable peritoneal dialysis treatment regimen in a step 107. The prescription can also further be optimized manually.

Finally, in step 108, a peritoneal dialysis treatment in accordance with the selected peritoneal dialysis treatment regimen is performed on the patient to be treated. To that end, the machine is supplied with one or more peritoneal dialysis solution bags containing the peritoneal dialysis solution or solutions for that particular treatment regimen, i.e. a peritoneal dialysis solution having the appropriate solution type and glucose concentration or allowing the mixing of the appropriate solution(s) and concentrations(s) that correspond(s) to the selected treatment regimen. The patient is connected to the peritoneal dialysis machine and the peritoneal dialysis machine is operated to apply a succession of treatment cycles in accordance with the selected treatment regimen, i.e. the pumps and valves are operated with a view of supplying and withdrawing peritoneal dialysis fluid defined by inflow volume, and glucose concentration of the respective cycles as well as the total cycle time, dwell time as defined by the schedule of first and second treatment cycles.

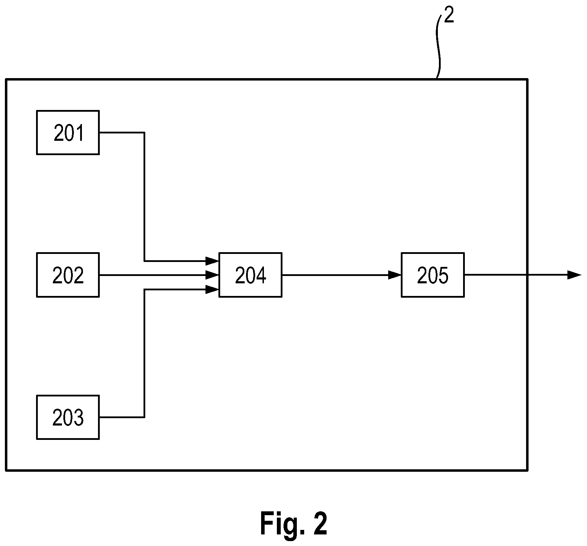

**Figure** 2 depicts a peritoneal dialysis regimen generating device 2 for generating a peritoneal dialysis regimen for controlling the operation of a peritoneal dialysis machine to operate to apply a succession of **first** exchange cycles and **second** exchange cycles within an allowable treatment parameter range/ therapy restriction(s) and thereby meet a therapy target or therapy target range. The regimen generating device 2 is adapted to be operated by applying the computer-implemented method 100 described in relation to figure 1.

The regimen generation device 2 includes a therapy target input unit 201 for inputting a therapy target or therapy target range for a particular patient. The therapy target may be inputted into a manually operated I/O device or inputted from a database assembly administrating patient data records comprising a therapy target or therapy target range as part of a respective patient data record. The therapy target or therapy target range may be prescribed by a physician.

The regimen generation device 2 further includes a patient characteristics input unit 202 for inputting patient characteristics. The patient characteristics may be inputted into a manually operated I/O device or inputted from a database assembly administrating patient data records comprising patient characteristics as part of a respective patient data record. Patient characteristics may include peritoneum transport characteristics and other patient characteristics as described above in relation to the computer-implemented method of figure 1.

The regimen generation device 2 further includes a peritoneal dialysis regimen generation unit 203 configured to generate an initial set of peritoneal dialysis regimens within the allowable treatment parameter range. Each peritoneal dialysis regimen within the initial set of peritoneal dialysis treatment regimens is defined by a succession of first cycles and second cycles, the first cycles being defined by a first inflow volume, a first dwell time and advantageously a first glucose concentration, the second cycles being defined by a second inflow volume, a second dwell time and advantageously a second glucose concentration, each treatment regimen within the initial set of peritoneal dialysis treatment regimens being defined by a number of first cycles and by a number of second cycles, the number of first cycles and/ or the number of second cycles being variable and/ or independent from each other among the plurality of generated peritoneal dialysis treatment regimens.

The peritoneal dialysis treatment regimen generation unit 203 may be configured to receive an allowable treatment parameter range input, wherein the treatment parameter range defines an interval of treatment parameters for the peritoneal dialysis regimens of the set of peritoneal dialysis treatment regimes. The allowable treatment parameter range/ therapy restriction(s) may be included in a patient data record of the patient to be treated. The allowable treatment parameter range/ therapy restriction(s) may be prescribed by a physician.

The therapy target input unit 201, the patient characteristics input unit 202 und the peritoneal dialysis regimen generation unit 203 are connected to the simulation unit 204 which is configured to simulate a peritoneal dialysis treatment outcome for each of the plurality of peritoneal dialysis treatment regimens to generate a simulated treatment outcome.

The simulation unit 204 is further adapted to match the simulated treatment outcome and the therapy target or therapy target range for the peritoneal dialysis treatment regimens within the set of peritoneal dialysis treatment regimens i.e. allowable treatment parameter range/ therapy restriction(s) to generate a set of treatment regimen candidates, that meet the predefined allowable treatment target or treatment target range.

The simulation unit 204 is connected to the selection unit 205 for transmission of the set of peritoneal dialysis treatment regimen candidates. In the selection unit 205 peritoneal dialysis treatment candidates of the set of peritoneal dialysis treatment candidates are made available for selection of the peritoneal dialysis treatment regimen to be applied for a specific treatment by an operator.

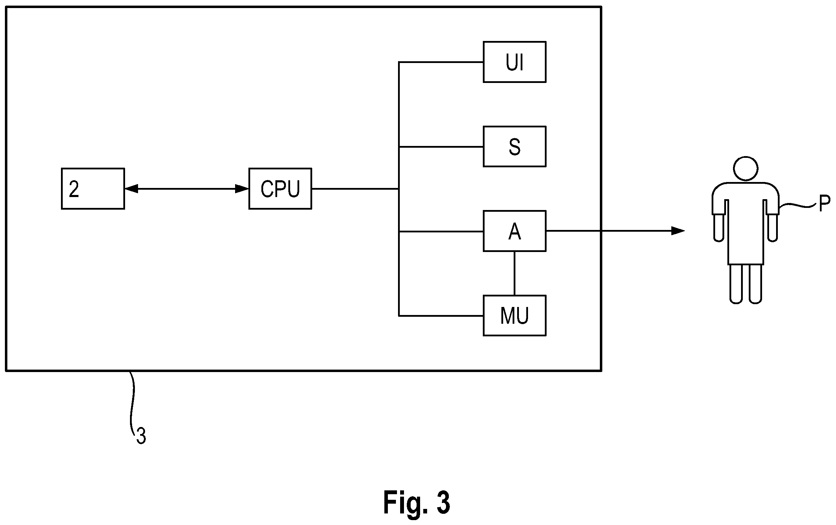

**Figure 3** depicts an embodiment of a peritoneal dialysis machine 3 comprising the the regimen generation device 2 described above in relation to figure 2. The peritoneal dialysis machine 3 comprises a user interface UI, at least one sensor S and an infusion unit A including one or more actors, such as valves and pumps that operate the peritoneal dialysis machine in accordance with a particular treatment regimen. The infusion unit A is fluidly connected to the patient P receiving peritoneal dialysis treatment to supply peritoneal dialysis solution to the patient and withdraw a solution comprising metabolic waste from the patient. For each of the cycles of the treatment regimen a certain solution concentration may be defined. In one embodiment a dialysis solution of a concentration of a predefined set of concentrations e.g 1.5 %, 2.3 % or 4.25 % glucose is applied for each cycle of the treatment regimen. Solution sources, such as solution bags e.g of 1.5 %, 2.3 % or 4.25 % glucose may be sequentially connected to the infusion unit A to infuse a dialysis solution of the appropriate concentration defined for a certain cycle of a treatment regimen to a patient.

In an alternative embodiment the infusion unit A is connected to a solution mixing unit MU for mixing a peritoneal dialysis solution from input solutions to create one or more peritoneal dialysis solution concentrations required for the peritoneal dialysis treatment regimen. The mixing of input concentrates may be performed within the solution mixing unit MU or within the peritoneal abdomen by sequentially infusing a solution of a first concentration and a solution of a second concentration into the patient's abdomen, the sequential infusing being controlled be the solution mixing unit. The solution mixing unit MU is connected to at least two solution sources, such as solution bags e.g of 1.5 %, 2.3 % or 4.25 % glucose. If sequential infusion and mixing with the patient's abdomen is applied, the inflow phase may be divided into two distinct inflow phases, each with a different solution (glucose). In this embodiment the glucose concentration for a cycle may be vary within an interval e.g. [1.5 %, 2.3%] or [2.3%, 4.25%] glucose, depending on the concentrations available for the solution sources. For example, a cycle with volume V and glucose concentration g (e.g. g having any value between 1.5% and 2.3% glucose) may be defined within a treatment regimen. Then two adjacent inflow phases are determined with volumes V1 and V2, and e.g. glucose concentrations g1 = 1.5 % and g2 ₌ 2.3 % (or g1 ₌ 2.3 % and g2 ₌ 4.25%), so that:

$$V1 + V2 = V$$

$$g1*V1 + g2*V2 = g*V$$

Given g1, g2, g and V, equations above can be solved to compute V1 and V2.

The solution mixing unit may be controlled to perform a sequence of two inflows (V1, g1) and (V2, g2) that will result (within the patient abdomen) in a volume V and an equivalent mixed glucose g.

This mixing of concentrates may be done for each cycle of the treatment regimen - wherein the only restriction is not to exceed the maximum number of bags that the solution mixing unit may handle (e.g. 6 bags).

The user interface UI, the sensor S, the infusion unit A and the regimen generation device 2 are connected to the central processing unit CPU. The central processing unit CPU receives the selected peritoneal dialysis treatment regimen for a particular treatment from the regimen generation device 2 and controls the infusion unit A and/or the solution mixing unit MU, to perform a peritoneal dialysis treatment on patient P by applying the fluid exchange cycles in accordance with the selected peritoneal dialysis treatment regimen.

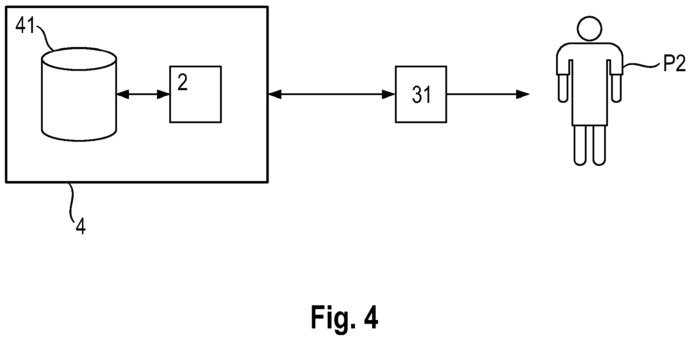

**Figure 4** depicts a peritoneal dialysis system 6 comprising a patient database assembly 4 for supplying a treatment regimen or a set of treatment regimens to a peritoneal dialysis device 31 fluidly connected to a patient P2 for applying a peritoneal dialysis treatment to the patient P2. The patient database assembly 4 comprises a database module 41 for administrating patient data records of peritoneal dialysis patients. Each patient data record may include a patient characteristics, an allowable treatment parameter range/ therapy restriction(s) and a therapy target or a therapy target range as described above. The database module 41 is connected to the regimen generation device 2 to provide patient characteristics and a therapy target or therapy target range to the regimen generation device as described above in relation to figure 2. The patient database assembly 4 is connected to the peritoneal dialysis machine 31, for transmitting a peritoneal dialysis regimen record. The peritoneal dialysis machine 31 is in fluidly connected to the patient P2 to perform the peritoneal treatment in accordance with the received dialysis treatment regimen, similarly as described above in relation to figure 3.

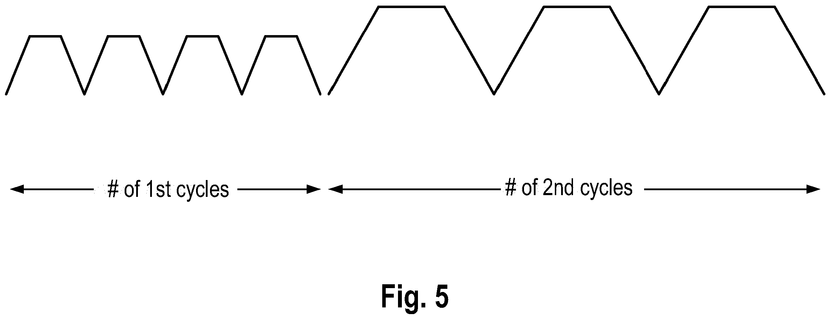

**Figure 5** is a graphical representation of a candidate set of peritoneal dialysis treatment regimens inputted into the simulation as described above.

In accordance with the embodiment depicted in figure 5, a number of first cycles precedes a number of second cycles, wherein the first cycles are smaller and shorter and the second cycles are larger and longer.

Typically the shorter and smaller cycles target ultrafiltration, whereas the longer and larger cycles target solute clearance. Advantageously, a first glucose concentration is defined for the cycles targeting ultrafiltration and a second glucose concentration is defined for the cycles targeting solute clearance, wherein a larger glucose concentration is defined for the cycles targeting ultrafiltration, thereby creating a larger osmotic gradient and/or osmotic pressure.

In accordance with the embodiment depicted in figure 5 the number of first, i.e. smaller and shorter cycles and the number of second cycles are variable and independent from each other among the plurality of generated peritoneal dialysis treatment regimens.

In the embodiment depicted in figure 5, the peritoneal dialysis treatment regimens have the following degrees of freedom: the number of first shorter and smaller cycles, the number of second longer and larger cycles and the solution type / glucose concentration(s) of the peritoneal dialysis solution(s) to be applied for the treatment. The parameter range of the glucose concentration may be sampled depending on the demands of the subsequent simulation step and/ or depending on available peritoneal dialysis solutions or PD machine capabilities.

In accordance with the embodiment of figure 5, inflow volume of the first and second cycles are determined according to a first and a second predefined relation to the body surface area, The dwell time and total cycle time of the first and second cycles may be each determined according to a corresponding predefined relation to the transporter type or peritoneum transport characteristics for the respective first or second cycles.

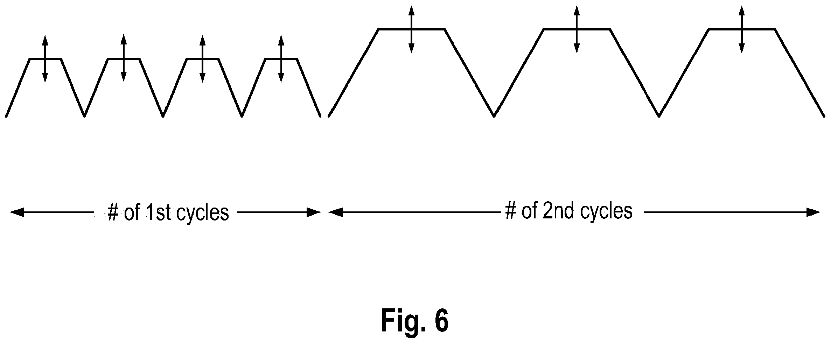

**Figure 6** is a graphical representation of a candidate set of peritoneal dialysis treatments in an alternative embodiment. Like in the embodiment depicted in figure 5, shorter and smaller cycles precede larger and longer cycles and the number of first, i.e. smaller and shorter cycles and the number of second, i.e. larger and longer cycles are variable and independent from each other among the plurality of generated peritoneal dialysis treatment regimens. Furthermore, the dwell time and total cycle time of the first and second cycles may be each determined according to a corresponding predefined relation to the transporter type or peritoneum transport characteristics for the respective first or second cycles. Different to the embodiment depicted in figure 5, the absolute volume of the small and/or the large exchange cycle is an additional degree of freedom. A fixed ratio may be defined between the volume of exchange volumes of small exchange cycles and the size of exchange volume of large exchange cycles. Alternatively, the exchange volumes of large and small exchange cycles may be varied independently. Thus, the parameter range or solution space has the following degrees of freedom: the volume of the small and/or the large exchange cycles, the number of small cycles, the number of large cycles, and the solution type /glucose concentration(s) of the peritoneal dialysis solution(s). The parameter range may be sampled according to simulation requirements and/ or solution type availability or mixing and/or solution profiling capabilities of the PD machine (for the glucose concentration).

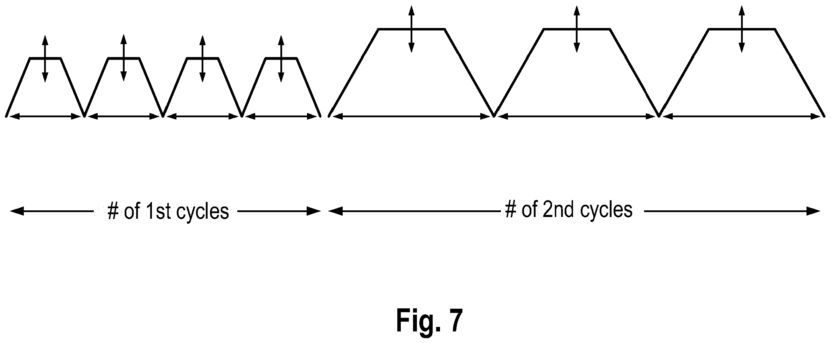

**Figure 7** is a graphical representation of a candidate set of peritoneal dialysis treatments in a further alternative embodiment. Like in the embodiments depicted in figure 5 and figure 6, shorter and smaller cycles precede larger and longer cycles and the number of first, i.e. smaller and shorter cycles and the number of second, i.e. larger and longer cycles are variable and independent from each other among the plurality of generated peritoneal dialysis treatment regimens.

Unlike the embodiments depicted in figure 5, and in figure 6, dwell times of the larger and/or the smaller exchange cycles are additional degrees of freedom. A fixed ratio may be defined between the dwell time of the short exchange cycles and the dwell time of the long exchange cycles. Alternatively, the dwell times of the short and the long exchange cycles may be varied independently.

Thus, the parameter range or solution space has the following degrees of freedom: the absolute volume of the small and/or the large exchange cycle, the dwell time of the short and/ or long exchange cycles, the number of small cycles, the number of large cycles, and the solution type/ glucose concentration(s) of the peritoneal dialysis solution(s). The parameter range may be sampled according to the demands of the subsequent simulation step, and/or available peritoneal dialysis solutions or mixing and/or solution profiling capabilities of the PD machine (for the glucose concentration.

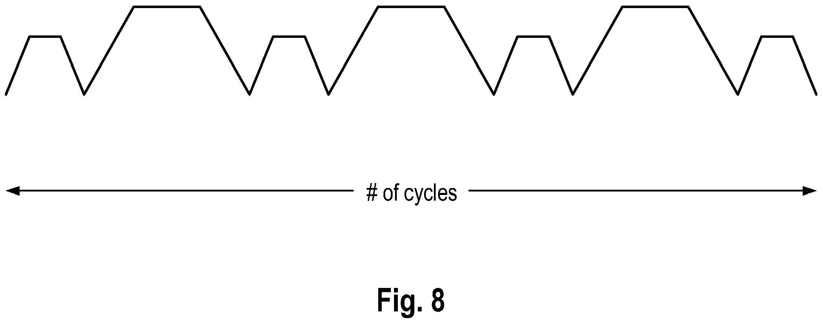

**Figure 8** is a graphical representation of an alternative candidate set of peritoneal dialysis treatment regimens inputted into the peritoneal dialysis treatment simulation. In accordance with the embodiment depicted in figure 8, smaller and shorter cycles of a first type alternate with larger and longer cycles of a second type.

In accordance with the embodiment depicted in figure 8 the number of first, i.e. smaller and shorter cycles and the number of second, i.e. larger and longer cycles are variable.

Therefore, in accordance with the embodiment depicted in figure 8, the number of shorter/smaller cycles and the number of larger/ longer cycles are coupled. In relation to the parameters fill volume, dwell time and glucose concentration as parameters or degrees of freedom of a peritoneal dialysis treatment regimen, the candidate set of peritoneal dialysis treatment regimens depicted in figure 8 corresponds to the candidate set of figure 5.

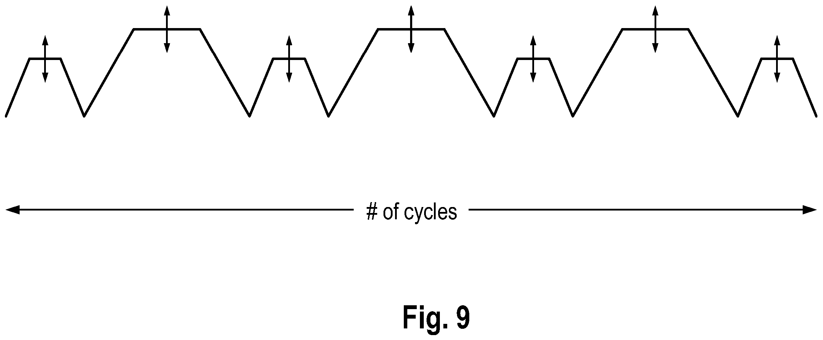

**Figure 9** is a graphical representation of a further alternative candidate set of peritoneal dialysis treatment regimens inputted into the peritoneal dialysis treatment simulation.

Similar to the candidate sets depicted in figure 8, smaller and shorter cycles of a first type alternate with larger and longer cycles of a second type, and the number of first, i.e. smaller and shorter cycles and the number of second, i.e. larger and longer cycles are variable. In relation to the parameters fill volume, dwell time and glucose concentration as parameters or degrees of freedom of a peritoneal dialysis treatment regimen, the candidate set of peritoneal dialysis treatment regimens depicted in figure 9 corresponds to the candidate set of figure 6.

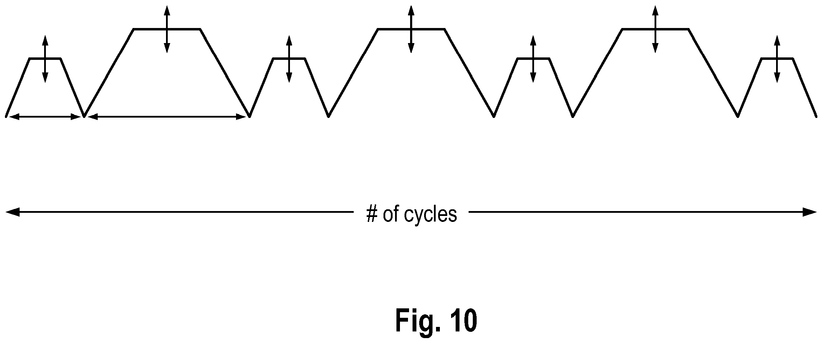

**Figure 10** is a graphical representation of a yet another candidate set of peritoneal dialysis treatment regimens to be simulated by the peritoneal dialysis treatment simulation.

Similar to the candidate set depicted in figure 8 and figure 9, first, smaller and shorter cycles alternate with second, larger and longer cycles, and the number of first and second cycles is variable. In relation to the parameters fill volume, dwell time and glucose concentration as parameters or degrees of freedom of a peritoneal dialysis treatment regimen, the candidate set of peritoneal dialysis treatment regimens depicted in figure 10 corresponds to the candidate set of figure 7.

**Claims**

1. Computer-implemented method (100) of generating a peritoneal dialysis regimen for controlling the operation of a peritoneal dialysis treatment machine to meet a therapy target or therapy target range, comprising:

   - receiving a therapy target input (101),
   - receiving a patient characteristics input (102),
   - generating a plurality of peritoneal dialysis treatment regimens (103), **characterized in that**
   - each peritoneal dialysis regimen is defined by:

     > a succession of first cycles and second cycles, wherein

       • the first cycles are respectively defined by a first inflow volume and a first dwell time, and wherein
       • the second cycles are respectively defined by a second inflow volume and a second dwell time,

     > a number of first cycles and by a number of second cycles, the number of first cycles and/or the number of second cycles being variable and/or independent from each other among the plurality of generated peritoneal dialysis treatment regimens,

   and **in that**
   the method (100) further comprises the following steps:

     - simulating a peritoneal dialysis treatment outcome (104) for each of the plurality of dialysis treatment regimens considering the patient characteristics input to generate a simulated treatment outcome, and

     - making available a peritoneal dialysis treatment regimen to be applied for a specific treatment for selection (106) based on the simulated treatment outcome that meets the therapy target input.

2. Computer-implemented method (100) for generating a peritoneal dialysis regimen according to claim 1, wherein a fixed ratio between first dwell times and second dwell times is predefined.

3. Computer-implemented method (100) for generating a peritoneal dialysis regimen according to any of the preceding claims, wherein a fixed ratio between first inflow volumes and second inflow volumes is predefined.

4. Computer-implemented method (100) for generating a peritoneal dialysis regimen according to any of the preceding claims, wherein the first cycles are shorter and smaller than the second cycles.

5. Computer-implemented method (100) for generating a peritoneal dialysis regimen according to claim 4, wherein a first glucose concentration is defined for the first cycles and a second glucose concentration is defined for the second cycles, and wherein the first glucose concentration is larger than the second glucose concentration.

6. Computer-implemented method (100) for generating a peritoneal dialysis regimen according to claim 3, wherein the first cycles precede the second cycles.

7. Computer-implemented method (100) for generating a peritoneal dialysis regimen according to claim 3, wherein the first cycles and the second cycles alternate each other.

8. Device (2) adapted to perform the computer-implemented method as defined in claims 1 to 7 for generating a peritoneal dialysis regimen for controlling the operation of a peritoneal dialysis treatment machine to meet a therapy target or therapy target range, the device (2) comprising:

   - a therapy target input unit (201),
   - a patient characteristics input unit (202),
   - a peritoneal regimen generation unit (203), configured to generate a plurality of peritoneal dialysis regimens, **characterised in that**

     each peritoneal dialysis regimen is defined by
     a succession of first cycles and second cycles, the first cycles being defined by a first inflow volume and a first dwell time, the second cycles being defined by a second inflow volume and a second dwell time, each treatment regimen being defined by a number of first cycles and by a number of second cycles, the number of first cycles and / or the number of second cycles being variable and/ or independent from each other, among the plurality of generated peritoneal dialysis treatment regimens and wherein the device (2) further comprises:
- a simulation unit (204), configured to simulate a peritoneal dialysis treatment outcome for each of the plurality of peritoneal dialysis treatment regimens to generate a simulated treatment outcome, and
- a selection unit (205), configured to select a peritoneal dialysis treatment regimen to be applied for a specific treatment from the plurality of peritoneal dialysis treatment regimens based on the respective simulated treatment outcome that meets the therapy target input.

9. Peritoneal dialysis treatment machine (3), comprising the device (2) for generating a peritoneal dialysis regimen defined in claim 8.

10. A patient database assembly (4) for supplying a treatment regimen or a set of treatment regimens, the patient database assembly (4) including the device (2) as defined in claim 8, connected to a database module (41) for administrating patient data records including therapy target input (101), and patient characteristics input (102) wherein said database module (41) is an interface between the regimen generating device (2) as defined in claim 8, and the patient database assembly (4).

11. Peritoneal dialysis system including the patient database assembly (4) as defined in claim 10, and a peritoneal dialysis treatment machine (31), connected to said patient database assembly (4) for downloading a peritoneal dialysis regimen from the patient database assembly (4), and adapted to operate in accordance with the downloaded peritoneal dialysis regime.

12. Computer program comprising instructions which, when being executed by a computer, cause the computer to execute a computer-implemented method according to one of claims 1 to 7.

13. Computer-readable medium comprising instructions for the execution of a computer-implemented method according to one of claims 1 to 7, when the instructions are executed on a computer.

**Patentansprüche**

1. Computerimplementiertes Verfahren (100) zum Erzeugen eines Peritonealdialyseplans zum Steuern des Betriebs einer Peritonealdialyse-Behandlungsmaschine derart, dass ein Therapieziel oder ein Therapiezielbereich erfüllt wird, umfassend:

> - Empfangen einer Therapiezieleingabe (101),
> - Empfangen einer Patientenkennwerteeingabe (102),
> - Erzeugen einer Vielzahl von Peritonealdialyse-Behandlungsplänen (103), **dadurch gekennzeichnet, dass**
> - jeder Peritonealdialyseplan durch Folgendes definiert ist:
>
>> > eine Folge von ersten Zyklen und zweiten Zyklen, wobei
>>
>>> • die ersten Zyklen jeweils durch ein erstes Zuflussvolumen und eine erste Verweilzeit definiert sind und wobei
>>> • die zweiten Zyklen jeweils durch ein zweites Zuflussvolumen und eine zweite Verweilzeit definiert sind,
>>
>> > eine Anzahl von ersten Zyklen und durch eine Anzahl von zweiten Zyklen, wobei die Anzahl von ersten Zyklen und/oder die Anzahl von zweiten Zyklen variabel und/oder unabhängig voneinander in der Vielzahl von erzeugten Peritonealdialyse-Behandlungsplänen sind,

und dadurch, dass das Verfahren (100) ferner die folgenden Schritte umfasst:

> - Simulieren eines Peritonealdialyse-Behandlungsergebnisses (104) für jeden der Vielzahl von Dialyse-Behandlungsplänen unter Berücksichtigung der Patientenkennwerteeingabe, um ein simuliertes Behandlungsergebnis zu erzeugen, und
> - Zur-Verfügung-Stellen eines Peritonealdialyse-Behandlungsplans, der für eine spezifische Behandlung angewendet werden soll, zur Auswahl (106) basierend auf dem simulierten Behandlungsergebnis, das die Therapiezieleingabe erfüllt.

2. Computerimplementiertes Verfahren (100) zum Erzeugen eines Peritonealdialyseplans nach Anspruch 1, wobei ein festes Verhältnis zwischen ersten Verweilzeiten und zweiten Verweilzeiten vordefiniert wird.

3. Computerimplementiertes Verfahren (100) zum Erzeugen eines Peritonealdialyseplans nach einem der vorhergehenden Ansprüche, wobei ein festes Verhältnis zwischen ersten Zuflussvolumen und zweiten Zuflussvolumen vordefiniert wird.

4. Computerimplementiertes Verfahren (100) zum Erzeugen eines Peritonealdialyseplans nach einem der vorhergehenden Ansprüche, wobei die ersten Zyklen kürzer und kleiner sind als die zweiten Zyklen.

5. Computerimplementiertes Verfahren (100) zum Erzeugen eines Peritonealdialyseplans nach Anspruch 4, wobei eine erste Glukosekonzentration für die ersten Zyklen definiert wird und eine zweite Glukosekonzentration für die zweiten Zyklen definiert wird und wobei die erste Glukosekonzentration größer ist als die zweite Glukosekonzentration.

6. Computerimplementiertes Verfahren (100) zum Erzeugen eines Peritonealdialyseplans nach Anspruch 3, wobei die ersten Zyklen den zweiten Zyklen vorausgehen.

7. Computerimplementiertes Verfahren (100) zum Erzeugen eines Peritonealdialyseplans nach Anspruch 3, wobei die ersten Zyklen und die zweiten Zyklen einander abwechseln.

8. Vorrichtung (2), eingerichtet zum Durchführen des computerimplementierten Verfahrens nach einem der Ansprüche 1 bis 7 zum Erzeugen eines Peritonealdialyseplans zum Steuern des Betriebs einer Peritonealdialyse-Behandlungsmaschine derart, dass ein Therapieziel oder ein Therapiezielbereich erfüllt wird, wobei die Vorrichtung (2) Folgendes umfasst:

- eine Einheit zur Therapiezieleingabe (201),
- eine Einheit zur Patientenkennwerteeingabe (202),
- eine Einheit zur Erzeugung von Peritonealdialyseplänen (203), die zum Erzeugen einer Vielzahl von Peritonealdialyseplänen konfiguriert ist,
**dadurch gekennzeichnet, dass**

jeder Peritonealdialyseplan definiert ist durch eine Folge von ersten Zyklen und zweiten Zyklen, wobei die ersten Zyklen durch ein erstes Zuflussvolumen und eine erste Verweilzeit definiert sind, die zweiten Zyklen durch ein zweites Zuflussvolumen und eine zweite Verweilzeit definiert sind, jeder Behandlungsplan definiert ist durch eine Anzahl von ersten Zyklen und durch eine Anzahl von zweiten Zyklen, wobei die Anzahl von ersten Zyklen und/oder die Anzahl von zweiten Zyklen variabel und/oder unabhängig voneinander in der Vielzahl von erzeugten Peritonealdialyse-Behandlungsplänen sind,
und wobei die Vorrichtung (2) ferner Folgendes umfasst:

- eine Simulationseinheit (204), die konfiguriert ist zum Simulieren eines Peritonealdialyse-Behandlungsergebnisses für jeden der Vielzahl von Peritonealdialyse-Behandlungsplänen, um ein simuliertes Behandlungsergebnis zu erzeugen, und
- eine Auswahleinheit (205), die konfiguriert ist zum Auswählen eines Peritonealdialyse-Behandlungsplans, der für eine spezifische Behandlung angewendet werden soll, aus der Vielzahl von Peritonealdialyse-Behandlungsplänen basierend auf dem jeweiligen simulierten Behandlungsergebnis, das die Therapiezieleingabe erfüllt.

9. Peritonealdialyse-Behandlungsmaschine (3), umfassend die Vorrichtung (2) zum Erzeugen eines Peritonealdialyseplans nach Anspruch 8.

10. Patientendatenbankanordnung (4) zum Liefern eines Behandlungsplans oder eines Satzes von Behandlungsplänen, wobei die Patientendatenbankanordnung (4) die Vorrichtung (2) nach Anspruch 8 einschließt, die an ein Datenbankmodul (41) zum Darreichen von Patientendatenaufzeichnungen, einschließlich einer Therapiezieleingabe (101) und einer Patientenkennwerteeingabe (102), angeschlossen ist, wobei das Datenbankmodul (41) eine Schnittstelle zwischen der Planerzeugungsvorrichtung (2) nach Anspruch 8 und der Patientendatenbankanordnung (4) ist.

11. Peritonealdialysesystem, die Patientendatenbankanordnung (4) nach Anspruch 10 und eine Peritonealdialyse-Behandlungsmaschine (31) einschließend, die an die Patientendatenbankanordnung (4) angeschlossen ist, um einen Peritonealdialyseplan von der Patientendatenbankanordnung (4) herunterzuladen, und zum Betrieb gemäß dem heruntergeladenen Peritonealdialyseplan eingerichtet ist.

12. Computerprogramm, Anweisungen umfassend, die bei Ausführung durch einen Computer den Computer veranlassen, ein computerimplementiertes Verfahren nach einem der Ansprüche 1 bis 7 auszuführen.

13. Computerlesbares Medium, Anweisungen zur Ausführung eines computerimplementierten Verfahrens nach einem der Ansprüche 1 bis 7, wenn die Anweisungen auf einem Computer ausgeführt werden, umfassend.

**Revendications**

1. Procédé mis en oeuvre par ordinateur (100) permettant de générer un schéma de dialyse péritonéale pour commander le fonctionnement d'une machine de traitement par dialyse péritonéale pour atteindre un objectif thérapeutique ou une plage d'objectifs thérapeutiques, comprenant :

   - la réception d'une entrée d'objectif thérapeutique (101),
   - la réception d'une entrée de caractéristiques du patient (102),
   - la génération d'une pluralité de schémas thérapeutiques par dialyse péritonéale (103), **caractérisé en ce que**
   - chaque schéma de dialyse péritonéale est défini par :

      > une succession de premiers cycles et de deuxièmes cycles, dans lequel

         • les premiers cycles sont respectivement définis par un premier volume d'apport et un premier temps de séjour, et dans lequel
         • les deuxièmes cycles sont respectivement définis par un deuxième volume d'apport et un deuxième temps de séjour,

      > un nombre de premiers cycles et par un nombre de deuxièmes cycles, le nombre de premiers cycles et/ou le nombre de deuxièmes cycles étant variables et/ou indépendants les uns des autres parmi la pluralité de schémas thérapeutiques par dialyse péritonéale générés,

   et **en ce que** : le procédé (100) comprend en outre les étapes suivantes consistant à :

      - simuler un résultat de traitement par dialyse péritonéale (104) pour chaque schéma de la pluralité de schémas thérapeutiques par dialyse en tenant compte de l'entrée des caractéristiques du patient pour générer un résultat de traitement simulé, et
      - rendre disponible un schéma thérapeutique par dialyse péritonéale à appliquer pour un traitement spécifique sélectionnable (106), compte tenu du résultat de traitement simulé qui répond à l'entrée d'objectif thérapeutique.

2. Procédé mis en oeuvre par ordinateur (100) permettant de générer un schéma de dialyse péritonéale selon la revendication 1, dans lequel un rapport fixe entre les premiers temps de séjour et les deuxièmes temps de séjour est prédéfini.

3. Procédé mis en oeuvre par ordinateur (100) permettant de générer un schéma de dialyse péritonéale selon l'une quelconque des revendications précédentes, dans lequel un rapport fixe entre des premiers volumes d'apport et des deuxièmes volumes d'apport est prédéfini.

4. Procédé mis en oeuvre par ordinateur (100) permettant de générer un schéma de dialyse péritonéale selon l'une quelconque des revendications précédentes, dans lequel les premiers cycles sont plus courts et plus petits que les deuxièmes cycles.

5. Procédé mis en oeuvre par ordinateur (100) permettant de générer un schéma de dialyse péritonéale selon la revendication 4, dans lequel une première concentration de glucose est définie pour les premiers cycles et une deuxième concentration de glucose est définie pour les deuxièmes cycles, et dans lequel la première concentration de glucose est supérieure à la deuxième concentration de glucose.

6. Procédé mis en oeuvre par ordinateur (100) permettant de générer un schéma de dialyse péritonéale selon la revendication 3, dans lequel les premiers cycles précèdent les deuxièmes cycles.

7. Procédé mis en oeuvre par ordinateur (100) permettant de générer un schéma de dialyse péritonéale selon la revendication 3, dans lequel les premiers cycles et les deuxièmes cycles alternent.

8. Dispositif (2) adapté à la réalisation du procédé mis en oeuvre par ordinateur défini dans les revendications 1 à 7 permettant de générer un schéma de dialyse péritonéale pour commander le fonctionnement d'une machine de traitement par dialyse péritonéale pour atteindre un objectif thérapeutique ou une plage d'objectifs thérapeutiques, le dispositif (2) comprenant :

      - une unité d'entrée d'objectif thérapeutique (201),
      - une unité d'entrée de caractéristiques du patient (202),
      - une unité de génération de schéma de dialyse péritonéal (203) configurée pour générer une pluralité de schémas de dialyse péritonéale, **caractérisé en ce que**

         chaque schéma de dialyse péritonéale est défini par une succession de premiers cycles et de deuxièmes cycles, les premiers cycles étant définis par un premier volume d'apport et un premier temps de séjour, les deuxièmes cycles étant définis par un deuxième volume d'apport et un deuxième temps de séjour, chaque schéma thérapeutique étant défini par un nombre de premiers cycles et un nombre de deuxièmes cycles, le nombre de premiers cycles et/ou le nombre de deuxièmes cycles étant variables et/ou indépendants les uns des autres, parmi la pluralité de schémas thérapeutiques par dialyse péritonéale générés,
et le dispositif (2) comprenant en outre :

- une unité de simulation (204) configurée pour simuler un résultat de traitement par dialyse péritonéale pour chaque schéma de la pluralité de schémas thérapeutiques par dialyse péritonéale pour générer un résultat de traitement simulé, et
- une unité de sélection (205) configurée pour sélectionner un schéma thérapeutique par dialyse péritonéale à appliquer pour un traitement spécifique parmi la pluralité de schémas thérapeutiques par dialyse péritonéale compte tenu du résultat de traitement simulé respectif qui répond à l'entrée d'objectif thérapeutique.

9. Machine de traitement par dialyse péritonéale (3) comprenant le dispositif (2) de génération de schéma de dialyse péritonéale défini dans la revendication 8.

10. Ensemble de base de données de patients (4) permettant de fournir un schéma thérapeutique ou un ensemble de schémas thérapeutiques , l'ensemble de base de données de patient (4) incluant le dispositif (2) défini dans la revendication 8, connecté à un module de base de données (41) pour administrer des dossiers de données de patients incluant une entrée d'objectif thérapeutique (101) et une entrée de caractéristiques du patient (102), ledit module de base de données (41) étant une interface entre le dispositif de génération de schéma (2) défini dans la revendication 8 et l'ensemble de base de données de patient (4).

11. Système de dialyse péritonéale incluant l'ensemble de base de données de patient (4) défini dans la revendication 10, et une machine de traitement par dialyse péritonéale (31) connectée audit ensemble de base de données de patient (4) pour télécharger un schéma de dialyse péritonéale à partir de l'ensemble de base de données de patient (4) et adaptée à un fonctionnement conforme au schéma de dialyse péritonéale téléchargé.

12. Programme informatique comprenant des instructions qui, lorsqu'elles sont exécutées par un ordinateur, amènent l'ordinateur à exécuter un procédé mis en oeuvre par ordinateur selon l'une des revendications 1 à 7.

13. Support lisible par ordinateur comprenant des instructions permettant l'exécution d'un procédé mis en oeuvre par ordinateur selon l'une des revendications 1 à 7 lorsque les instructions sont exécutées sur un ordinateur.

| receive a therapy target input | —101 |

| receive a patient characteristics input | —102 |

| receive an allowable treatment parameter range input | —109 |

| generate a plurality of peritoneal dialysis regimens | —103 |

| simulate peritoneal dialysis treatment to generate simulated treatment outcome | —104 |

| match therapy target input with the simulated treatment outcome | —105 |

| make peritoneal dialysis treatment available for selection based on simulated treatment outcome | —106 |

| operator selects treatment regimen | —107 |

| treatment regimen applied to patient | —108 |

100

**Fig. 1**

Fig. 2

Fig. 3

**Fig. 4**

**Fig. 5**

Fig. 6

# of 1st cycles    # of 2nd cycles

**Fig. 7**

EP 3 638 338 B1

# of cycles

**Fig. 8**

# of cycles

**Fig. 9**

# of cycles

**Fig. 10**

**EP 3 638 338 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20110009810 A1

- WO 2010006131 A2

### Non-patent literature cited in the description

- Kinetic modelling in Peritoneal Dialysis. **GOTCH ; KEEN.** Clinical Dialysis. 2005, 385-419